# EUROPEAN PATENT SPECIFICATION

(11) **EP 4 164 749 B1**
(45) Date of publication and mention of the grant of the patent: **07.08.2024**
(21) Application number: 21730946.7
(22) Date of filing: 08.06.2021
(51) Int. Cl.: A61P 31/14, A61K 9/08, A61K 47/20, A61K 31/05, A61K 45/06

(54) **COMPOUND AND METHOD FOR THE TREATMENT OF CORONAVIRUSES**
VERBINDUNG UND VERFAHREN ZUR BEHANDLUNG VON CORONAVIREN
COMPOSÉ ET PROCÉDÉ POUR LE TRAITEMENT DES CORONAVIRUS

(30) Priority: 10.06.2020 EP 20305633; 26.10.2020 EP 20306279; 12.05.2021 EP 21305623
(43) Date of publication of application: 19.04.2023
(73) Proprietor: Apteeus, 59000 Lille (FR); Université de Lille, 59800 Lille (FR); Centre National de la Recherche Scientifique, 75016 Paris (FR); Institut National de la Santé et de la Recherche Médicale, 75013 Paris (FR); Centre Hospitalier Universitaire de Lille, 59000 Lille (FR)
(72) Inventor: BEGHYN, Terence, 59320 Haubourdin (FR); DEPREZ, Benoît, 59000 Lille (FR); BELOUZARD, Sandrine, 59130 Lambersart (FR); BRODIN, Priscille, 59700 Marcq-en-Baroeul (FR)
(74) Representative: Cabinet Beau de Loménie
(86) International application number: PCT/EP2021/065356
(87) International publication number: WO 2021/250038

(56) References cited:
- EP-A1- 3 459 537
- WO-A1-2007/009264
- WO-A1-2017/042636
- HOFFMANN MARKUS ET AL: "SARS-CoV-2 Cell Entry Depends on ACE2 and TMPRSS2 and Is Blocked by a Clinically Proven Protease Inhibitor", CELL, ELSEVIER, AMSTERDAM NL, vol. 181, no. 2, 5 March 2020 (2020-03-05), pages 271, XP086136225, ISSN: 0092-8674, [retrieved on 20200305], DOI: 10.1016/J.CELL.2020.02.052

## Description

### Field of the invention

The present invention relates to the use of clofoctol for the treatment of a disease caused by a coronavirus, in particular Covid-19.

### Background of the invention

Coronaviruses are belonging to the family *Coronaviridae* (order *Nidoviraies*) and include viruses with a single-strand, positive-sense RNA genome approximately 26-32 kilobases in size. The *Coronaviridae* family includes Alphacoronavirus (alphaCoV), Betacoronavirus (betaCoV), Deltacoronavirus (deltaCoV) and Gammacoronavirus (gammaCoV). Bats and rodents are thought to be the reservoir for alphaCoV and betaCoV. Currently, it is less clear which animals serve as the reservoir for deltaCoV and gammaCoV. Coronaviruses are named according to their appearance under the electron microscope, the viruses look like they are covered with pointed structures that surround them like a corona or crown due to the presence of spike glycoproteins on their envelope.

Three coronaviruses have crossed the species barrier to cause deadly pneumonia in humans since the beginning of the 21^{st} century: Severe Acute Respiratory Syndrome coronavirus (SARS-CoV), Middle East Respiratory Syndrome coronavirus (MERS-CoV), and SARS-CoV-2 (also known as 2019-nCoV). SARS-CoV-2 is an enveloped, positive-sense, single-stranded RNA virus which belongs to the betaCoV genus, which genus also includes SARS-CoV and MERS-CoV. SARS-CoV-2 shares 89% nucleotide identity with bat SARS-like CoV and 82% identity with human SARS-CoV. Other coronaviruses which can infect man include human coronavirus NL63 (HcoV-NL63), human coronavirus OC43 (HcoV-OC43), human coronavirus HKU1 (HcoV-HKU1) and human coronavirus 229E (HcoV-229E). Coronavirus entry into host cells is mediated by the transmembrane spike (S) glycoprotein that forms homotrimers protruding from the viral surface. It has been shown that in addition to direct membrane fusion, SARS-CoV could enter cells through an endocytic route, and this endocytic infection leads to viral gene expression (Cell Research 2008; 18: 290-301). In respect of SARS-CoV-2, it has been suggested that ACE2 (angiotensin-converting enzyme 2) mediates SARS-CoV-2 S-mediated entry into cells, establishing it as a functional receptor for this newly emerged coronavirus; SARS-CoV-2 S engages human ACE2 with comparable affinity to SARS-CoV S (Cell 2020; 180: 281-292). It has also been shown that SARS-CoV-2 uses transmembrane protease, serine 2 (TMPRSS2) for S protein priming (Cell 2020; 181: 271-280).

Since the emergence of SARS-CoV-2, and the outbreak of the associated disease, Covid-19, a number of drugs, either investigational or already on the market, have been tested for their potential efficacy in treating / alleviating the effects of Covid-19. To the knowledge of the inventors, none of these drugs have been shown to block the virus cycle when both entry pathways of SARS-CoV-2 are available in the cells, i.e. the endocytic entry and the fusion entry, at concentrations that can be reached in the infected tissue at a tolerable posology.

Clofoctol (2-(2,4-Dichlorobenzyl)-4-(1,1,3,3-tetramethylbutyl)phenol, CAS number 37693-01-9, PubChem CID 2799) is a bacteriostatic antibiotic, indicated for the treatment of infections caused by Gram-positive bacteria. It was first described in French patent application 2 101 076. This compound has subsequently been shown to activate the unfolded protein response pathways, making it a potential drug candidate for the treatment of prostate cancer (British Journal of Pharmacology 2014; 171: 4478-4489). More recently, clofoctol has been show to activate EBV lytic gene expression (Journal of Virology 2019; 93(20): e00998-19) and to suppress glioma stem cell proliferation by activating KLF13 (J Clin Invest. 2019; 129(8):3072-3085).

### Summary of the invention

It has now been found that clofoctol blocks the virus cycle when both entry pathways of SARS-CoV-2 are available in the cells, i.e. the endocytic entry and the fusion entry. As a consequence, clofoctol has been found to be effective in suppressing the cytopathic effect induced by SARS-CoV-2, and in reducing the viral load in cells infected with the virus. Accordingly, the invention relates to clofoctol for use in the prevention or treatment of a disease caused by a coronavirus such as SARS-CoV, MERS-CoV, SARS-CoV-2, HCoV-NL63, HCoV-OC43, HCoV-HKU1 or HCoV-229E. The invention also relates to a method of treating a disease caused by a coronavirus, such as SARS-CoV, MERS-CoV, SARS-CoV-2, HCoV-NL63, HCoV-OC43, HCoV-HKU1 or HCoV-229E, which method comprises administering clofoctol to a subject in need thereof.

### Brief description of the figures

Figure 1 and Figure 3 show the efficacy of various concentrations of clofoctol on the cytopathic effect of SARS-CoV-2 infecting Vero81 cells, not transduced (Fig.1) or transduced (Fig.3) with the TMPRSS2 gene.
Figure 2 and Figure 4 show the efficacy of various concentrations of clofoctol on growth improvement of Vero81 cells, not transduced (Fig.2) or transduced (Fig.4) with the TMPRSS2 gene, and infected with SARS-CoV-2.
Figure 5 and Figure 6 show the effect of clofoctol on the genomic replication of SARS-CoV-2 in Vero81 cells and in Vero81 cells transduced with TMPRSS2, infected with SARS-CoV-2.
Figure 7 shows the effect of clofoctol on the SARS-CoV-2 load in Vero81 cells and in Vero81 cells transduced with the TMPRSS2 gene, infected with SARS-CoV-2.
Figure 8 shows the effect of clofoctol on the genomic replication of SARS-CoV-2 in Calu-3 cells infected with SARS-CoV-2.
Figure 9 shows pharmacokinetics of clofoctol in C57BL/6J mice.
Figure 10 shows the effect of clofoctol on SARS-CoV-2 infection in K18-hACE2 transgenic C57BL/6J mice.
Figure 11 shows the effect of clofoctol on the genomic replication of SARS-CoV-2 in Vero81 cells infected with two different variants of SARS-CoV-2.
Figure 12 shows the effect of clofoctol on the inhibition of HCoV-229E-Rluc virus growth in Huh-7 cells.
Figure 13 shows the effect of clofoctol on viral secretion in infected Vero81 cells (lower curve) and infected Vero81 cells transduced with TMPRSS2 (upper curve).
Figure 14 shows the effect of clofoctol on viral replication in infected Vero81 cells (upper curve) and infected Vero81 cells transduced with TMPRSS2 (lower curve).
Figure 15 shows the effect of clofoctol on viral secretion in infected human bronchial epithelia.
Figure 16 shows the effect of clofoctol on viral secretion in infected human bronchial epithelia.

### Detailed description of the invention

In the context of the present disclosure, the various embodiments described hereafter can be combined.

In a first aspect, the present invention relates to clofoctol for use in the prevention or treatment of a disease caused by a coronavirus such as SARS-CoV, MERS-CoV, SARS-CoV-2, HCoV-NL63, HCoV-OC43, HCoV-HKU1 or HCoV-229E. In particular, clofoctol is particularly suitable for the prevention or treatment of a disease caused by a coronavirus such as SARS-CoV, MERS-CoV, SARS-CoV-2.

In one embodiment, the coronavirus is SARS-CoV-2, and the corresponding disease is Covid-19. It will be appreciated by a person skilled in the art that variants of SARS-CoV-2 are encompassed by the generic term "SARS-CoV-2". Examples of such variants include the B. 1 variant with a D614G mutation, from which the B. 1. 1.7 variant, also known as the "British" variant, descends; the B.1.526 variant first identified in New York City; the B.1.351 variant also known as the "South African" variant;, the B.1.1.248 variant also known as the "Brazilian" variant; the B.1.427 and B.1.429 variants identified notably in California; the variant B.1.617 also known as the "Indian" variant..

In one embodiment, clofoctol is administered in combination with at least one other active principle (small molecule or biological) which is or has been under investigation for the treatment of SARS-CoV, MERS-CoV or SARS-CoV-2. The administration of the at least one other active principle can be simultaneous, sequential or over a period of time, with respect to the administration of clofoctol.

In one embodiment the at least one other active principle is selected from:
Abemaciclib, almitrine bismesylate, amodiaquine, anakinra, angiotensin 1-7, anidulafungin, apixaban, aspirin, avatrombopag, azithromycin, bamlanivimab, baricitinib, bazedoxifene, berbamine, brexpiprazole, bromhexine, camostat, canakinumab, casirivimab, cepharanthine, ceritinib, cetylpyridinium, chloroquine, chlorpromazine, ciclesonide, clomifene citrate, cobicistat, croconazole, cyclosporine, danoprevir, darunavir, dexamethasone, digitoxin, digoxin, dihydroartemisinine, dihydrogambogic acid, diltiazem, dronedarone, drotaverine, ebastine, eltrombopag, emapalumab, etesevimab, ethaverine, favipiravir, fingolimod, flunarizine, gilteritinib, harringtonine, hematoporphyrin, hexachlorophene, hydrocortisone, hydroxychloroquine, hydroxyprogesterone caproate, imdevimab, IMU-838, interferon Beta-1A, interferon Beta-1B, isoosajin, isopomiferin, isotretinoin, ivacaftor, ivermectin, JS016, ketoconazole, lidoflazine, loperamide, lopinavir, loratadine, loteprednol etabonate, lusutrombopag, LY-CoV555, mefloquine, methylprednisolone, molnupiravir, nafamostat, niclosamide, nitazoxanide, omacetaxine mepesuccinate, osajin, oseltamivir osimertinib, otilimab, ouabain, oxiconazole, oxyclozanide, ozanimod, papaverine, pegylated interferon lambda, perhexiline maleate, pexidartinib, PF-07321332, phenazopyridine, polydocanol, posaconazole, prednisone, recombinant human interferon α1β, recombinant Interferon Alfa-2b, regorafenib, remdesivir, ribavirin, ritonavir, ruxolitinib, sitagliptin, sofosbuvir, sonidegib, sorafenib, tamoxifen, thalidomide, thimerosal, thioridazine, thymosin alpha 1, tilorone, tioguanine, tocilizumab, toremifene, triparanol, tyrphostin, umifenovir.

In one embodiment, clofoctol is administered in the form of a pharmaceutical composition comprising at least one pharmaceutically acceptable excipient (in addition to clofoctol). Excipients, pharmaceutical compositions and method for their preparation are well known in the art (see e.g. Handbook of pharmaceutical Excipients, Rowe et al, seventh edition, June 2012; Rules and Guidance For Pharma Manufacturers and distributors 205, Medecines and Healthcare products Regulatory Agency, London UK).

In another aspect, the present invention relates to a method of preventing or treating a disease caused by a coronavirus, such as SARS-CoV, MERS-CoV, SARS-CoV-2, HCoV-NL63, HCoV-OC43, HCoV-HKU1 or HCoV-229E, which method comprises administering clofoctol to a subject in need thereof. In some embodiments the coronavirus is SARS-CoV, MERS-CoV or SARS-CoV-2. In some embodiments, the coronavirus is SARS-CoV-2, and the corresponding disease is Covid-19.

The current route of administration of clofoctol is the rectal route. Suppositories containing 100 mg, 200 mg or 750 mg of clofoctol can be prepared with suitable pharmaceutically acceptable excipients.

In one embodiment the subject has one or more of the following conditions or diseases: overweight, obesity, diabetes, hyperlipidaemia, arterial hypertension, cardiovascular disease, chronic kidney disease, immunosuppression.

In one embodiment, the method further comprises the administration to the subject of at least one other active principle. The administration of the at least one other active principle can be simultaneous, sequential or over a period of time, with respect to the administration of clofoctol.

In one embodiment the at least one other active principle is as defined above.

Clofoctol has been shown to suppress the cytopathic effect of hSARS-CoV2 in Vero81 cells as well as in Vero81 cells transduced with TMPRSS2 and in human Calu-3 cells. Vero81 cells are permissive to the virus with an entry way based on endocytosis. When transduced with TMPRSS2, Vero81 cells become permissive to the virus with 2 entry ways, endocytosis and fusion. Clofoctol has also been shown to inhibit the viral genomic replication in the same cells and in human Calu-3 cells that also offer both entries for the virus. Clofoctol has further been shown to reduce the viral load of cell cultures infected with the virus (Vero81 cells and Vero81 cells transduced with TMPRSS2). Clofoctol has further been shown to reduce the virus load in the lungs of SARS-CoV-2 infected mice (K18-hACE2). Clofoctol has further been shown to inhibit the viral genomic replication of 2 different variants of SARS-CoV-2. Clofoctol has further been shown to inhibit the replication of HCoV-229E, which is an alpha-coronavirus.

The following examples have been included for the purpose of illustration, and are not to be construed as limiting in any manner.

### Examples

### Virus isolation & amplification

Human coronavirus SARS-CoV2 (hSARS-CoV2) was isolated from patient samples and annotated BetaCoV/France/IDF0372/2020. It was used *in vitro* and amplified on VERO-81 cells expressing TMPRSS2, cultivated on plates and incubated until the complete destruction of cell monolayers. SARS-CoV-2 of lineage B1.1.7 (GISAID accession number EPI_ISL_1653931) is a second variant of SARS-CoV-2 used *in vitro.* After harvesting, virus titrations were performed by serial 10-fold dilutions of supernatants (10⁻¹ to 10⁻⁸) distributed onto VERO-81 cells incubated with DMEM supplemented with 2% FBS (Fetal Bovine Serum). The plates were incubated for 5 days at 37 °C with 5% CO₂. Afterwards, the plates were examined using an inverted microscope (ZEISS Primovert) to evaluate the extent of the virus-induced cytopathic effect in cell culture. Calculation of estimated virus concentration was carried out by the Spearman and Karber method and expressed as log10 TCID₅₀/mL (50% tissue culture infectious dose, i.e. the amount of a virus that produces a cytopathic effect in 50% of the infected cells). The limit of detection of the method was 1.5 TCID₅₀/ml.

The hCoV-19_IPL_France strain of SARS-CoV-2 (NCBI MW575140) was also used in *in vitro* and *in vivo* experiments. The virus was propagated in Vero-E6 cells expressing TMPRSS2 by inoculation at MOI 0.01. Cell supernatant medium was harvested at 72 hours post-infection and stored frozen at -80 °C in small aliquots.

### TMPRSS2 transduction

Vero-81 cells were transduced with a lentivirus expressing TMPRSS2. TMPRSS2 was cloned into pTRIP vector, lentivectors were produced by transfection of HEK293T cells with pTRIP-TMPRSS2, phCMV-VSVG and HIV gag-pol with Turbofect^{™} (ThermoFischer) according to the manufacturer's instruction. Supernatants containing lentiviral vectors were used to transduce cells. Then cells were used in experiments 72h after the transduction.

### Measure of the cytopathic effect

### Cell culture:

Vero cells [ATCC^{®} CCL-81^{™}, ECACC, Sigma-Aldrich, France, hereafter "Vero81" cells] were cultured in Dulbecco's modified Eagle medium (DMEM) supplemented with 10% fetal bovine serum (FBS) and GlutaMAX^{™} in a humidified atmosphere of 5% CO₂ at 37 °C. Huh-7 cells were grown at 37°C with 5% CO2 in Dulbecco's modified eagle medium (DMEM, Gibco) supplemented with 10 % heat-inactivated fetal bovine serum (FBS, Eurobio).

Calu-3 cells (Clinisciences, EP-CL-0054) were grown in minimum essential medium (Gibco, MEM) supplemented with glutamax (Gibco) and 10% heat-inactivated FBS.

Vero-E6 cells (ATCC, CRL-1586) were grown at 37°C with 5% CO₂ in Dulbecco's modified eagle medium (DMEM, Gibco) supplemented with 10% heat-inactivated fetal bovine serum (FBS, Eurobio).

Tested compound: solutions of the clofoctol were prepared in DMSO at concentrations required to achieve the desired concentrations in the experiment. Solutions were dispensed onto the living cells. The maximum concentration of DMSO was 0.15%.

Cell infection: cells were infected after addition of clofoctol at various concentrations or control solvents. The multiple of infection was 0.01.

Reagents: Hoechst 33342, Trihydrochloride, trihydrate, (Ref H3570, ThermoFischer) and Propidium Iodide (Ref P1304MP, ThermoFisher) were respectively used at 10 µg/ml and 1 µg/ml respectively to monitor the viral cytopathic effect (CPE) of clofoctol. They were both added after an incubation time of 72h.

Results were obtained by analyzing the percentage of PI-positive nuclei, surrogate of the toxicity of the virus exerts on cells, and number of nuclei corresponding to the cell growth inhibition exerted by the virus.

### Measure of the viral genomic replication

Cell culture: Vero81 cells and Vero-81 cells transduced with TMPRSS2 were cultured in Dulbecco's modified Eagle medium (DMEM) supplemented with 10% fetal bovine serum (FBS) and GlutaMAX^{™} in a humidified atmosphere of 5% CO₂ at 37 °C.

Tested compound: solutions of clofoctol were prepared in DMSO at concentrations required to achieve the desired concentrations in the experiment. Solutions were dispensed onto the living cells. The maximum concentration of DMSO was 0.15%.

Cell infection: cells were infected after addition of clofoctol at various concentrations or control solvents. The multiple of infection was 0.25.

Results were obtained by dosing viral RNA related to total cellular RNA, surrogate of the viral loads.

RNA dosing: after 6 hours of incubation, total cellular RNA was extracted by using the Nucleospin RNA kit (Macherey-Nagel) as recommended by the manufacturer and SARS-CoV-2 genome were measured by quantitative RT-PCR. RNA was subjected to reverse transcription by using the high-capacity cDNA reverse transcription kit (Applied Biosystems). Then real time PCR was performed with TaqMan universal PCR master mix (Applied Biosystems) in a QuantStudio 3. Primers and probe (WHO protocol) targeting the open reading frame for the E gene were used. Standard curves were created by using in vitro transcribed RNA corresponding to the amplicon.

### Determination of viral loads in vitro

Vero-81 and Vero-81-TMPRSS2 cells were infected at a MOI of 0.25 for 1h, then the cells were rinsed 3 times with PBS and further incubated in the presence of increasing concentrations of clofoctol for 16h. Each condition was performed in duplicates. Cell supernatants were collected and viral titer were measured by the TCID₅₀ method.

### Determination of viral loads in the lungs of mice

To determine the viral loads in lungs, half of right lobes were homogenized in Lysing Matrix D tubes (mpbio) containing 1 mL of PBS using Mixer Mill MM 400 (Retsch) (15min - 15 Hz). After centrifugation at 11,000 rpm for 5 min, the clarified supernatant was harvested for virus titration. Dilutions of the supernatant were done in DMEM with 1% penicillin/streptomycin and dilutions were transferred to Vero-E6 cells in 96-well plates for TCID₅₀ assay.

### Assessment of gene expression by RTqPCR

Half of the right lobe was homogenized in 1 mL of RA1 buffer from the NucleoSpin RNA kit containing 20 mM of TCEP. Total RNAs in the tissue homogenate were extracted with NucleoSpin RNA from Macherey Nagel. RNAs were eluted with 60 µL of water.

RNA was reverse-transcribed with the High-Capacity cDNA Archive Kit (Life Technologies, USA). The resulting cDNA was amplified using SYBR Green-based real-time PCR and the QuantStudio^{™} 12K Flex Real-Time PCR Systems (Applied Biosystems^{™}, USA) following manufacturers protocol. Relative quantification was performed using the gene coding glyceraldehyde 3-phosphate dehydrogenase (*Gapdh*). Specific primers were designed using Primer Express software (Applied Biosystems, Villebon-sur-Yvette, France) and ordered to Eurofins Scientifics (Ebersberg, Germany). Relative mRNA levels (2^{ΔΔCt}) were determined by comparing (a) the PCR cycle thresholds (Ct) for the gene of interest and the house keeping gene (ΔCt) and (b) ΔCt values for treated and control groups (ΔΔCt). Data were normalized against expression of the *gapdh* gene and are expressed as a fold-increase over the mean gene expression level in mock-treated mice. Viral load is expressed as viral RNA normalized to *Gapdh* expression level (ΔCt).

### Example 1

The effect of clofoctol on Vero81 cells infected with hSARS-CoV2 was assessed by adding a range of concentrations of clofoctol, following 1:3 dilutions, prior to infection with hSARS-CoV2 at MOI 0.01 (Multiple Of Infection (MOI) 0.01 means that the amount of virus used is 0.01 per 1 cell) for 72 hours. Biological duplicates with two technical replicates were performed.

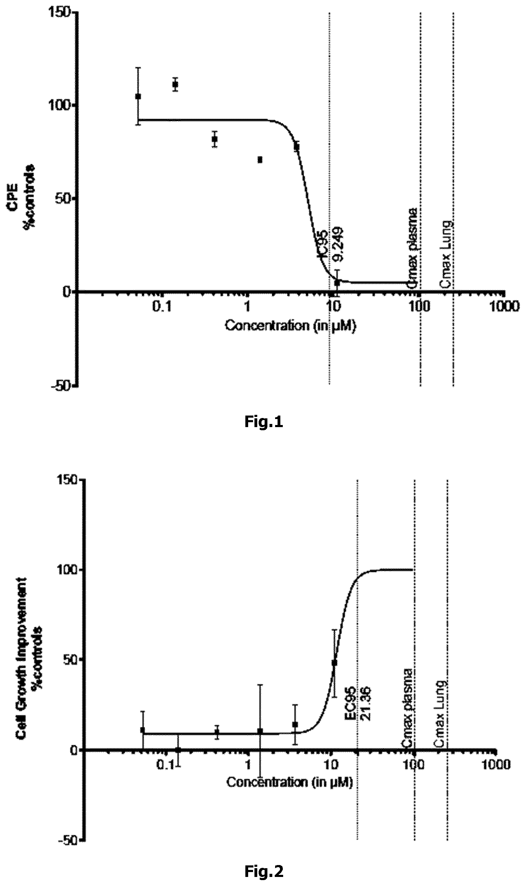

The results were obtained by analyzing the percentage of PI-positive nuclei expressed as the percentage of CPE relative to a scale defined by positive (0% infected cells) and negative (100% infected cells) controls. It can be seen from Figure 1 that clofoctol has a concentration reducing 95% (IC₉₅) of the CPE of 9.2µM.

### Example 2

The effect of clofoctol on Vero81 cells infected with hSARS-CoV2 was assessed by adding a range of concentrations of clofoctol, following 1:3 dilutions, prior to infection with hSARS-CoV2 at MOI 0.01 for 72 hours. Biological duplicates or triplicates with three technical replicates were performed.

The results were obtained by analyzing the number of living cells expressed as the percentage of cell growth improvement relative to a scale defined by normal cell growth (not infected controls) and no cell growth (infected controls). It can be seen from Figure 2 that the concentration of clofoctol at which 95% of its maximum response is observed (EC₉₅) is estimated at 21µM.

### Example 3

The effect of clofoctol on TMPRSS2-transduced Vero81 cells infected with hSARS-CoV2 was assessed by adding a range of concentrations of clofoctol, following 1:3 dilutions, prior to infection with hSARS-CoV2 at MOI 0.01 for 72 hours. Biological triplicates were performed.

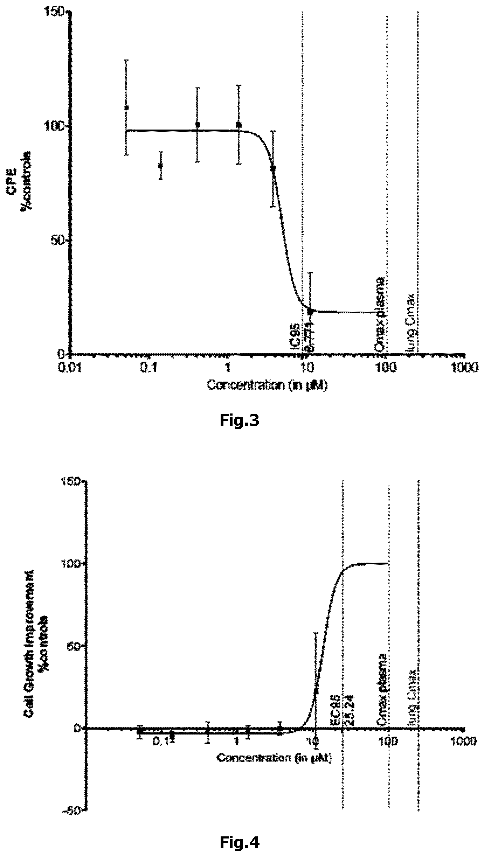

The results were obtained by analyzing the percentage of PI-positive nuclei expressed as the percentage of CPE relative to a scale defined by positive (0% infected cells) and negative (100% infected cells) controls. It can be seen from Figure 3 that clofoctol has an IC₉₅ of 8.7µM.

### Example 4

The effect of clofoctol on TMPRSS2-transduced Vero81 cells infected with hSARS-CoV2 was assessed by adding a range of concentrations of clofoctol, following 1:3 dilutions, prior to infection with hSARS-CoV2 at MOI 0.01 for 72 hours. Biological triplicates were performed.

The results were obtained by analyzing the number of living cells expressed as the percentage of cell growth improvement relative to a scale defined by normal cell growth (not infected controls) and no cell growth (infected controls). It can be seen from Figure 4 that clofoctol has an estimated EC₉₅ of 25µM.

### Example 5

The effect of clofoctol on Vero81 and TMPRSS2-transduced Vero81 cells infected with hSARS-CoV2 was assessed by adding a range of concentrations of clofoctol, following 1:2,5 dilutions, prior to infection with hSARS-CoV2 at MOI 0.25 for 6 hours. Biological duplicates were performed.

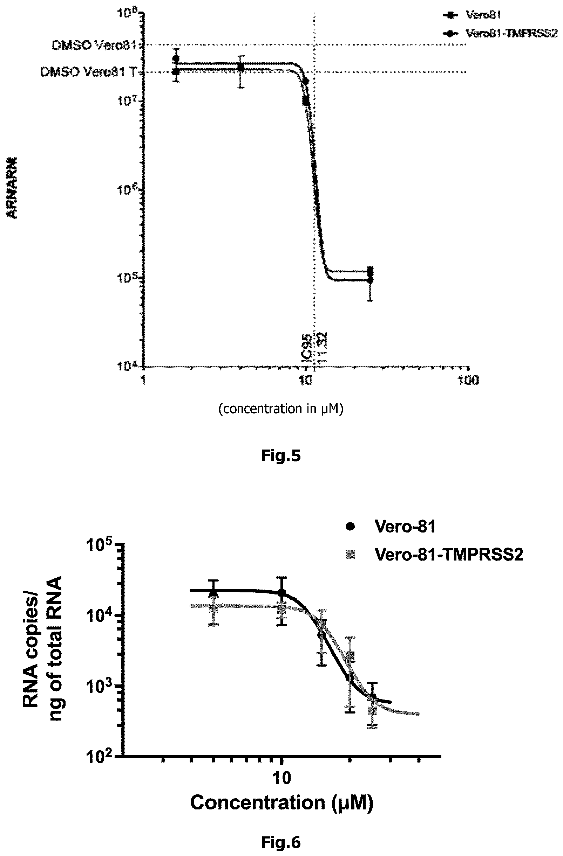

Results were obtained by dosing viral RNA and the viral load was expressed as the ratio of the viral RNA on the total RNA (RNAt) of the sample. It can be seen from Figure 5 that clofoctol has an IC₉₅ of 11µM.

The results in Examples 1-5 show that the IC₉₅ of clofoctol varies from about 9µM to about 11µM, i.e. values which are about 25 times less than the Cmax (about 250µM) reported for clofoctol in human lung tissue of patients, undergoing pulmonary resection or pneumonectomy, to which clofoctol was administered before or during surgery (Journal of Antimicrobial Chemotherapy 1987; 19: 679-683). Likewise, the EC₉₅ of clofoctol varies from about 21µM to about 25µM, i.e. values which are about 10 times less that the Cmax reported in human lung tissue (see above).

### Example 6

Vero-81 and Vero-81-TMPRSS2 cells were infected for 6h at a MOI of 0.25 in the presence of increasing concentrations of clofoctol. Then, total RNA was extracted and viral RNA was quantified by RT-qPCR. Results were normalized by the amount of total RNA and represent the average of three independent experiments performed in duplicates. The results are presented on Figure 6 from which it can be seen that clofoctol inhibits the genomic replication of SARS-CoV-2.

### Example 7

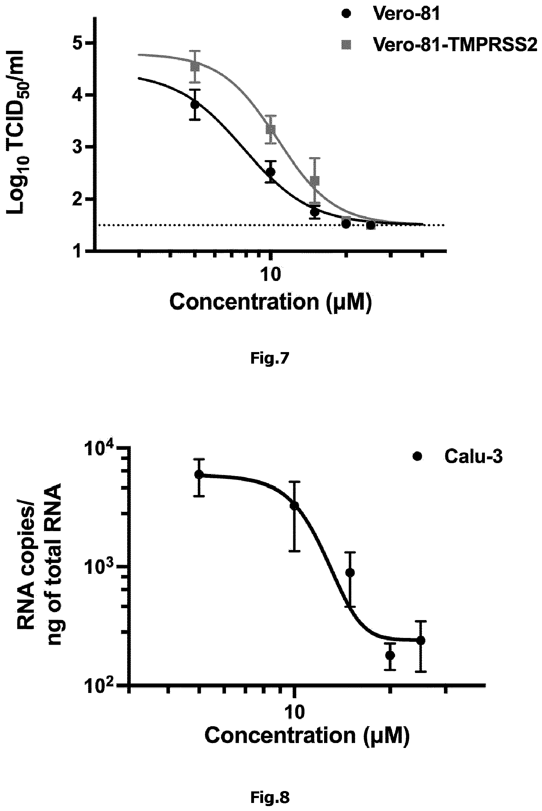

Vero-81 and Vero-81-TMPRSS2 cells were infected with SARS-CoV-2 at a MOI of 0.25. After 1h, the inoculum was removed and the cells were washed with PBS prior treatment with clofoctol. Cells were then further incubated for 16h. Thereafter, supernatants were collected and the amounts of secreted infectious virus were quantified. The dotted-line represents the limit of detection (1.5 TCID₅₀/mL). These data represent the average of three independent experiments (N=3). Experiments were performed in duplicate for each condition. The results are presented on Figure 7 from which it can be seen that the SARS-CoV-2 load decreases as the concentration of clofoctol increases.

### Example 8

Calu-3 cells were infected at a MOI of 0.25 in the presence of increasing concentrations of clofoctol for 24h. Then total cellular RNA was extracted and viral RNA was quantified by RT-qPCR. The results are presented on Figure 8 from which it can be seen that clofoctol inhibits SARS-CoV-2 replication in Calu-3 cells.

The results in examples 6-8 show that clofoctol displays an antiviral activity against Sars-CoV2.

### Example 9

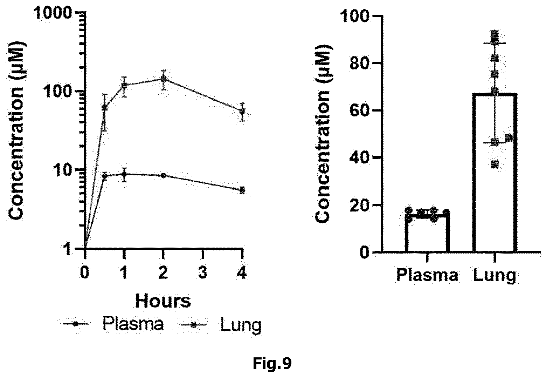

Clofoctol diluted in 1.75% final Kolliphor^{®} RH40 (07076, Sigma) and 1.4% final ethanol in a sodium chloride solution was used for intraperitoneal injection. Female 8-10 week-old C57BL/6J mice were treated i.p. with a single dose of clofoctol (62.5mg/kg) and were sacrificed at different time points thereafter (Figure 9, left panel). In a separate experiment, the mice were treated i.p. twice daily with a dose of clofoctol (62.5mg/kg) for two days, and the mice were sacrificed 1h after the last injection (Figure 9, right panel). The concentration of clofoctol in the plasma and lung of treated mice was measured as follows: plasma samples and lung tissues were collected and treated with absolute ethanol, in a ratio of 1 to 10 and 1 to 50, respectively. Lung tissues were homogenized with a mechanical lysis system (Tissue Lyzer II). Supernatants were obtained by centrifugation before injection in LC-MS/MS. Samples were analysed using UPLC system Acquity I Class (Waters), combined with a triple quadrupole mass spectrometer Xevo TQD (Waters). The column, placed at 40°C, was an Acquity BEH C8 50*2.1mm, 1.7µm column (Waters) and the following mobile phases were used: 5mM ammonium formate pH 3.75 in water, as solvent (A) and 5 mM ammonium formate pH 3,75 in acetonitrile as solvent (B). The results shown on Figure 9 represent the average of three independent experiments performed in triplicate (lung) or duplicate (plasma). It can be seen that clofoctol is distributed in lungs at very significant levels.

### Example 10

Eight week-old female K18-human ACE2 expressing C57BL/6 mice (B6.Cg-Tg(K18-hACE2)2Prlmn/J) were purchased from the Jackson Laboratory. For infection, mice were anesthetized by intraperitoneal injection of ketamine (100 mg/kg) and xylazine (10 mg/kg) and then intranasally infected with 50 µl of DMEM containing (or not, in a mock sample) 5×10² TCID₅₀ of hCoV-19 IPL_France strain of SARS-CoV-2 (NCBI MW575140). All experiments complied with current national and institutional regulations and ethical guidelines. The protocols were approved by the institutional ethical committee "Comité d'Ethique en Experimentation Animale" (CEEA) 75, Nord Pas-de-Calais (France).

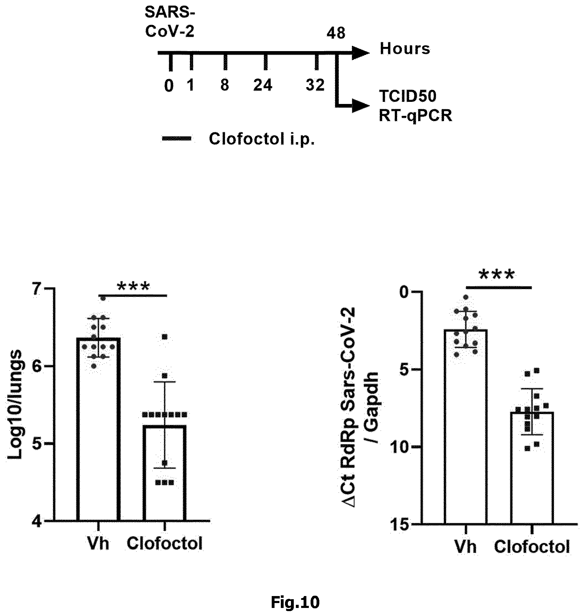

Mice were treated i.p. with clofoctol (62.5mg/kg) or vehicle 1h and 8h after i.n. inoculation of SARS-CoV-2 (5×10² TCID₅₀ per mouse) and twice daily at day 1 post-infection (Figure 10, left panel). Animals were sacrificed at day 2 and day 4 post-infection. The viral load was determined by titration on Vero-81 cells (Figure 10, middle panel) and by RT-qPCR (Figure 10, right panel) (day 2 post-infection).

It can be seen that clofoctol treatment significantly reduces SARS-CoV-2 infection in mice.

### Example 11

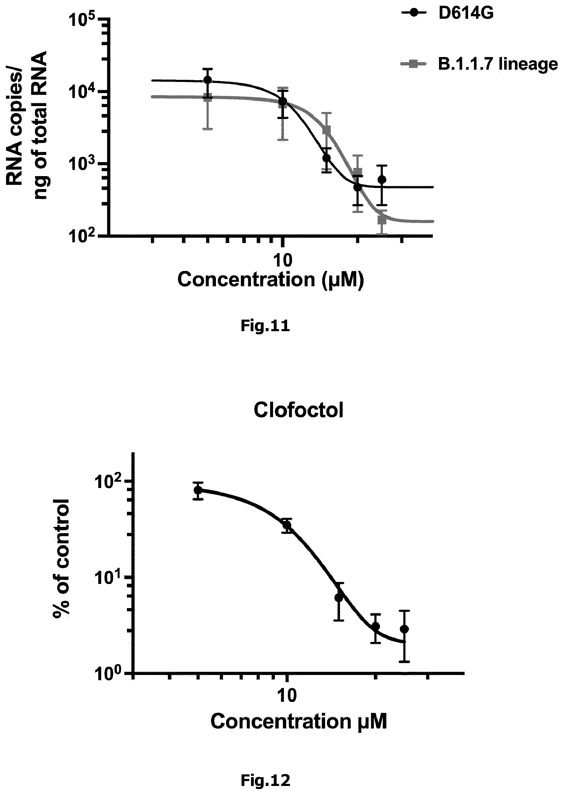

Vero-81 cells were infected either with SARS-CoV-2 of lineage B1 containing the D614G mutation (SARS-CoV-2/human/FRA/Lille_Vero-TMPRSS2/2020) or with SARS-CoV-2 of lineage B1.1.7 (GISAID accession number EPI_ISL_1653931). Viral genomes were quantified by RT-qPCR. Results were normalized by the amount of total RNA and represent the average of three independent experiments performed in duplicates. It can be seen from Figure 11 that clofoctol inhibits the genomic replication of variants of SARS-CoV-2.

### Example 12

Huh-7 cells were infected with HCoV-229E-Rluc in presence of different concentration of clofoctol. At 7h post-infection, cells were lysed and luciferase activities were quantified. The results are shown in Figure 12 and are presented as the percentage of the control and represent an average of three independent experiments performed in triplicates. Errors bars represent the standard error of the mean (SEM). It can be seen from Figure 12 that clofoctol is active against HCoV-229E, which is a different coronavirus from Sars-CoV2.

The above data in examples 1-12 evidence the value of clofoctol for the prevention or treatment of diseases caused by coronaviruses, such as Covid-19.

### Example 13

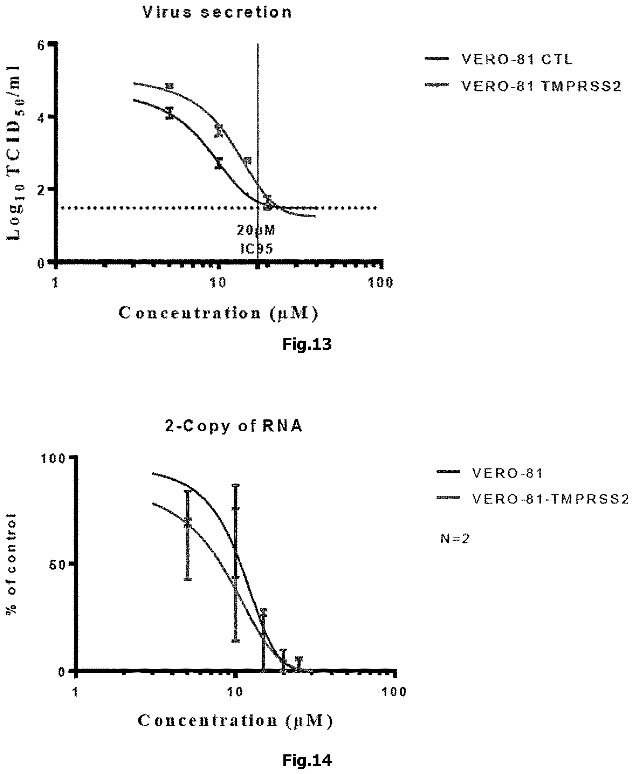

Vero81 cells and Vero-81 cells transduced with TMPRSS2 were infected with hSARS-CoV-2 at a MOI of 0.25 in DMEM medium with 10% foetal bovine serum. After 1 hour, the inoculum was removed and the cells were washed with PBS prior to treatment at different concentrations of clofoctol. Cells were then incubated in a humidified atmosphere of 5% CO₂ at 37°C for 16h. Thereafter, supernatants were collected and the quantity of secreted virus within was assessed by TCID₅₀ and expressed as log10 TCID₅₀/mL. The data reported in Figure 13 represent the average of two different experiences (n=2). Each condition was done in duplicate. The dotted-line in the figure represents the limit of detection of the method which is 1.5 TCID₅₀/ml.

It can be seen from Figure 13 that clofoctol inhibits the secretion of hSARS-CoV-2 by infected cells.

### Example 14

Vero81 cells and Vero-81 transduced with TMPRSS2 were infected with hSARS-CoV-2 at a MOI of 0.25 in DMEM medium with 10% foetal bovine serum in the presence of different concentrations of clofoctol. After 6 hours, the inoculum was removed and the cells were lysed to allow for total RNAs extraction using the Nucleospin RNA kit (Macherey-Nagel) and following the manufacturer's instructions. The SARS-CoV-2 genome was measured by quantitative RT-PCR using primers and probe (WHO protocol) targeting the open reading frame for the E gene. The results were normalized by the total quantity of RNA of each sample, and are expressed as a percentage of untreated control as shown in Figure 14. The experiment was done twice (n=2). Within each experiment, each condition was done in duplicate.

It can be seen from Figure 14 that clofoctol inhibits the replication of hSARS-CoV2 in infected cells.

### Example 15

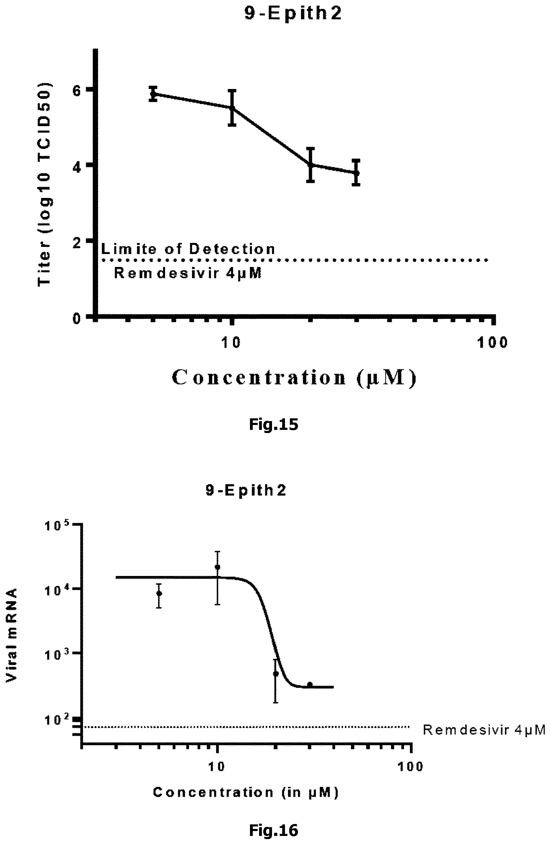

Human bronchial epithelia (MucilAir^{™}, available from the company Epithelix) were treated through apical administration of clofoctol. The epithelia were first washed with Mucilair^{®} culture medium containing different concentrations of clofoctol prior to infection by hSARS-CoV-2 at MOI 0.2 for 1 hour, still in the presence of clofoctol. Then, the inoculum was removed and epithelia were washed with PBS before addition at the apical pole of 25 µl of Mucilair^{®} culture medium containing different concentrations of clofoctol. After 72 hours of incubation at 37°C, 200 µl of MucilAir^{™} culture medium was added at the apical pole of the epithelia and harvested, after incubating for 5 minutes. These supernatants were used to quantify the amount of secreted virus as assessed by TCID₅₀. The results are shown in Figure 15 in which the dotted line represents the limit of detection of remdisivir. In addition, total RNAs were extracted by using the Nucleospin RNA kit (Macherey-Nagel) prior to genome quantification by RT-qPCR. The results could not be normalized due to the small amount of total RNA available and are shown in Figure 16. The data in both Figures 15 and 16 represent the average and standard-deviation of technical triplicates (n=3).

It can be seen from Figures 15 and 16 that clofoctol reduces the virus titer measured in the mucus of human bronchial epithelium in vitro by a factor more than 100 at 30µM. It is confirmed by the decrease of viral RNA quantitation.

## Claims

1. Clofoctol for use in the prevention or treatment of a disease caused by a coronavirus.

2. Clofoctol for the use of claim 1, wherein the coronavirus is SARS-CoV, MERS-CoV or SARS-CoV2.

3. Clofoctol for the use of claim 1, wherein the disease is Covid-19.

4. Clofoctol for the use of any preceding claims, wherein clofoctol is administered in combination with at least one other active principle.

5. Clofoctol for the use of claim 4, wherein the at least one other active principle is administered simultaneously, sequentially or over a period of time.

6. Clofoctol for the use of any preceding claims, wherein clofoctol is administered in the form of a pharmaceutical composition comprising at least one pharmaceutically acceptable excipient.

## Patentansprüche

1. Clofoctol zur Verwendung bei der Vorbeugung oder der Behandlung einer durch ein Coronavirus verursachten Krankheit.

2. Clofoctol zur Verwendung nach Anspruch 1, wobei das Coronavirus SARS-CoV, MERS-CoV oder SARS-CoV2 ist.

3. Clofoctol zur Verwendung nach Anspruch 1, wobei die Krankheit Covid-19 ist.

4. Clofoctol zur Verwendung nach einem der vorhergehenden Ansprüche, wobei Clofoctol in Kombination mit mindestens einem anderen Wirkstoff verabreicht wird.

5. Clofoctol zur Verwendung nach Anspruch 4, wobei der mindestens eine andere Wirkstoff gleichzeitig, nacheinander oder über einen bestimmten Zeitraum hinweg verabreicht wird.

6. Clofoctol zur Verwendung nach einem der vorhergehenden Ansprüche, wobei Clofoctol in Form einer pharmazeutischen Zusammensetzung verabreicht wird, die mindestens einen pharmazeutisch akzeptablen Hilfsstoff enthält.

## Revendications

1. Clofoctol pour utilisation dans la prévention ou le traitement d'une maladie causée par un coronavirus.

2. Clofoctol pour utilisation selon la revendication 1, où le coronavirus est SARS-CoV, MERS-CoV ou SARS-CoV2.

3. Clofoctol pour utilisation selon la revendication 1, où la maladie est le Covid-19.

4. Clofoctol pour utilisation selon l'une des revendications précédentes, où le clofoctol est administré en association avec au moins un autre principe actif.

5. Clofoctol pour utilisation selon la revendication 4, où le au moins un autre principe actif est administré simultanément, séquentiellement ou sur une période donnée.

6. Clofoctol pour utilisation selon l'une des revendications précédentes, où le clofoctol est administré sous la forme d'une composition pharmaceutique comprenant au moins un excipient pharmaceutiquement acceptable.
